# EUROPEAN PATENT SPECIFICATION

(11) **EP 2 874 551 B1**
(45) Date of publication and mention of the grant of the patent: **16.08.2017**
(21) Application number: 14768929.3
(22) Date of filing: 14.03.2014
(51) Int. Cl.: A61B 17/66

(54) **ORTHOPEDIC COMPRESSION/DISTRACTION DEVICE**
ORTHOPÄDISCHE KOMPRESSIONS-/DISTRAKTIONSVORRICHTUNG
DISPOSITIF ORTHOPÉDIQUE DE COMPRESSION/DISTRACTION

(30) Priority: 14.03.2013 US 201361782759 P
(43) Date of publication of application: 27.05.2015
(60) Divisional application: 15169035.1
(73) Proprietor: Wright Medical Technology, Inc., Memphis, TN 38117 (US)
(72) Inventor: THOREN, Brian, Memphis, TN 38122 (US); MCCORMICK, Daniel, Bartlett, TN 38133 (US); REED, Wesley, Libertyville, IL 60048 (US); CRAMER, Thomas, Gainesville, FL 32605 (US); LOWERY, Gary, Eads, TN 38028 (US); HARNESS, David, Eads, TN 38028 (US)
(74) Representative: Hirsch & Associés
(86) International application number: PCT/US2014/028641
(87) International publication number: WO 2014/153008

(56) References cited:
- KR-B1- 100 391 252
- US-A1- 2006 247 645
- US-A1- 2006 247 649
- US-A1- 2006 247 649
- US-B1- 6 245 071
- US-B2- 6 793 655

## Description

### FIELD OF THE INVENTION

The present disclosure relates to an orthopedic device for compression or distraction of bone parts.

### BACKGROUND

Orthopedic devices utilizing elongated pins as fasteners for compression or distraction of bone parts finds many uses for treating orthopedic patients. "Elongated pins" will be used herein to refer to various pins and wires, such as K-wires, used for fixating bone parts or providing anchors. Therefore, there is a continuing need for an improved orthopedic device that expands the scope and ability of the orthopedic surgeons in treating patients in a variety of conditions.

Furthermore, document US-A1-2006/0247649 is directed to an apparatus for use in orthopedic distraction or compression having two arms connected by a rod with a mechanism that enables the arms to be brought toward or away from each other.

### SUMMARY

The invention is directed to an orthopedic device according to claim 1.

### BRIEF DESCRIPTION OF THE DRAWINGS

**FIG. 1** is an illustration of an orthopedic device according to the present disclosure showing the locking sleeve attachments swiveled toward each other while the orthopedic device is in a distraction mode.
**FIG. 2A** is an illustration of the orthopedic device of FIG. 1 with the locking sleeve attachments removed.
**FIG. 2B** is an illustration showing the rack-and-pinion mechanism of the orthopedic device of FIG. 1.
**FIG. 3** is an illustration of the orthopedic device of FIG. 1 with 3-point bending yoke attachments.
**FIG. 4A** is an illustration of a biaxially swiveling locking sleeve attachment in its straight position viewed along its x-axis.
**FIG. 4B** is an illustration of the biaxially swiveling locking sleeve swiveled about the x-axis.
**FIG. 5A** is an illustration of the biaxially swiveling locking sleeve swiveled about its y-axis.
**FIG. 5B** is an illustration of the biaxially swiveling locking sleeve swiveled about the x-axis and the y-axis.
**FIG. 6A** is an illustration of the locking sleeve connected to the biaxial hinge block viewed along the γ-axis showing the plane K-K extending through the center of the locking sleeve along which the cross-sectional view of FIG. 6B is taken.
**FIG. 6B** is a cross-sectional view along K-K taken through the biaxially swiveling locking sleeve attachment in its swiveling position.
**FIG. 7A** is the illustration of FIG. 6A showing the plane L-L extending off-center through the locking sleeve along which the cross-sectional view of FIG. 7B is taken.
**FIG. 7B** is a cross-sectional view along L-L taken through the biaxially swiveling locking sleeve attachment in its swiveling position.
**FIG. 8A** is the illustration of FIG. 6A showing the plane M-M extending through the center of the locking sleeve along which the cross-sectional view of FIG. 8B is taken.
**FIG. 8B** is a cross-sectional view along M-M taken through the biaxially swiveling locking sleeve attachment in its non-swiveling position.
**FIG. 9A** is the illustration of FIG. 6A showing the plane N-N extending off-center through the locking sleeve along which the cross-sectional view of FIG. 9B is taken.
**FIG. 9B** is a cross-sectional view along N-N taken through the biaxially swiveling locking sleeve attachment in its swiveling position.
**FIG. 10A** is an illustration of the biaxially swiveling locking sleeve in its non-swiveling position shown with only the γ-axis locking pin portion of the biaxial hinge block shown.
**FIG. 10B** is an illustration of the biaxially swiveling locking sleeve in its swiveling position shown with only the γ-axis locking pin portion of the biaxial hinge block shown.
**FIG. 11A** is an illustration of the biaxially swiveling locking sleeve in the same configuration as in FIG. 10A viewed from a different angle.
**FIG. 11B** is an illustration of the biaxially swiveling locking sleeve in the same configuration as in FIG. 10B viewed from a different angle.
**FIG. 12** is an exploded orthographic view showing the threaded collet end of a locking sleeve and a collet nut where the collet nut is shown in cross-section.

The features shown in the above referenced drawings are illustrated schematically and are not intended to be drawn to scale nor are they intended to be shown in precise positional relationship. Like reference numbers indicate like elements.

### DETAILED DESCRIPTION

This description of the exemplary embodiments is intended to be read in connection with the accompanying drawings, which are to be considered part of the entire written description. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

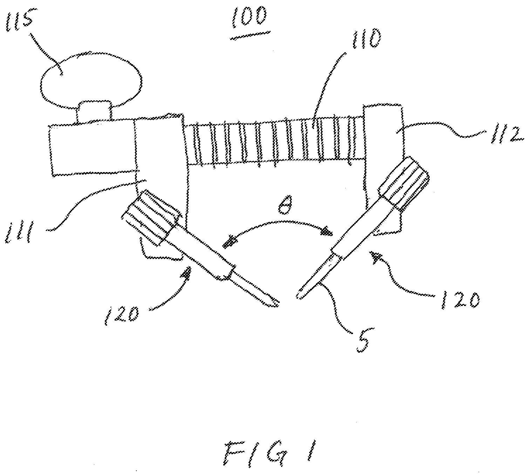

FIGS. 1-2B show an orthopedic device **100** that can be used for compression or distraction of bone parts according to an aspect of the present disclosure. The orthopedic device **100** comprises an elongated body **110** having first end **10** and a second end **20.** A first arm member **112** extends away from the first end **10** in a direction transverse to the elongated body **110.** The first arm member **112** can be integrally formed with the body **110** or otherwise attached to the elongated body **110.** A second arm member **111** transversely extends away from the elongated body **110.** The second arm member **111** is configured with a base portion **113** that is configured and adapted to movably engage the elongated body **110** allowing the second arm member **111** to be longitudinally movable along the elongated body to accomplish compression or distraction function of the device.

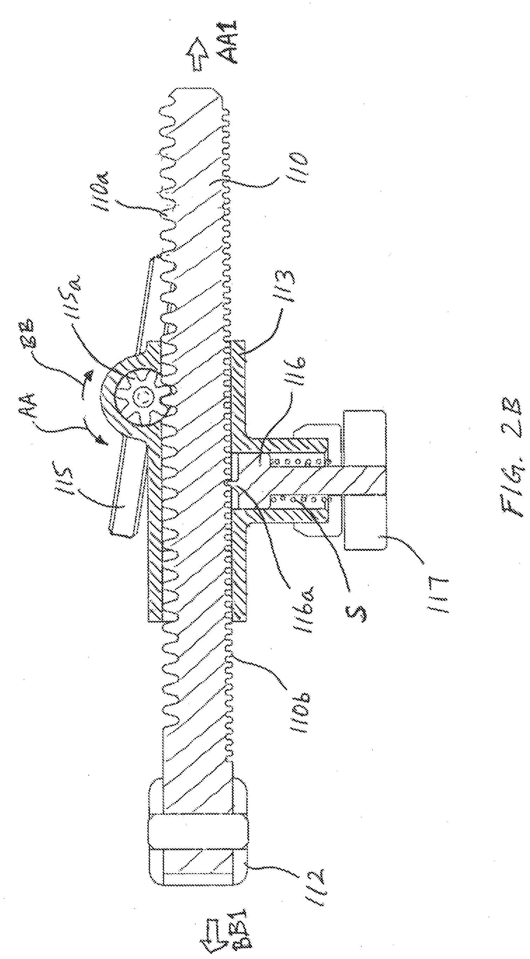

The actual structural mechanism for enabling the movable engagement between the elongated body **110** and the second arm member **111** can be one of many such structures known in the art. Referring to FIG. 2B, in one embodiment, the elongated body **110** is a rack of gear teeth **110a** and a pinion gear **115a** provided on the base portion **113** engages the rack of gear teeth **110a** for longitudinally moving the second arm member **111** along the elongated body. The pinion gear **115a** is rotatably attached to the base portion **113** and the second arm member **111** is moved longitudinally by turning the pinion gear **115a.** The pinion gear **115a** is provided with a bow **115,** similar to a bow on a key, to enable a user to turn the pinion gear.

The base portion **113** is configured with ratcheting mechanisms that can selectably operate in compression or distraction mode. For example, the base portion **113** can be provided with a spring-loaded ratcheting pin mechanism **116** for limiting the moving direction of the second arm member **111** along the elongated body **110** to be a one-way movement, as shown in FIG. 2B.

In the configuration shown in FIG. 2B, the spring-loaded ratcheting pin mechanism **116** is arranged so that the ratcheting pin mechanism **116** engages the ratchet teeth **110b** by a detent **116a**. The coil spring **S** urges the detent **116a** against the ratchet teeth **110b.** The detent **116a** has a slanted surface on one side as shown so that the ratchet teeth **110b** can depress the detent **116a** against the spring **S** and, thus, gliding over the detent **116a.** The elongated body **110** can move in the direction of arrow **AA1** by turning the pinion gear **115a** in the direction of the arrow **AA.** The detent **116a** will prevent the elongated body **110** from moving in the opposite direction shown by the arrow **BB1.**

In order to move the elongated body **110** in the direction of the arrow **BB1,** the spring-loaded ratcheting pin mechanism **116** is turned 180 degrees so that the slanted side of the detent **116a** is now facing in the opposite direction. This feature is used to change the direction of the one-way movement second arm member **111** so that the operation of the orthopedic device **100** is changed from compression to distraction and vice versa. The spring loaded ratcheting pin mechanism **116** can be turned 180 degrees by pulling the pin mechanism out using the thumb wheel **117,** turning it 180 degrees and releasing it. The spring bias will return the pin mechanism **116** to the seated position but with the detent **116a** now facing 180 degrees from before. Then, the pinion gear **115a** can be turned in the direction of the arrow **BB** and move the elongated body **110** in the direction of the arrow **BB1.** Another example of the ratcheting mechanism between the base portion **113** and the elongated body **110** is described in United States patent Application Serial No. 13/712,300, filed by the Applicant on December 12, 2012, the contents of which is incorporated herein by reference.

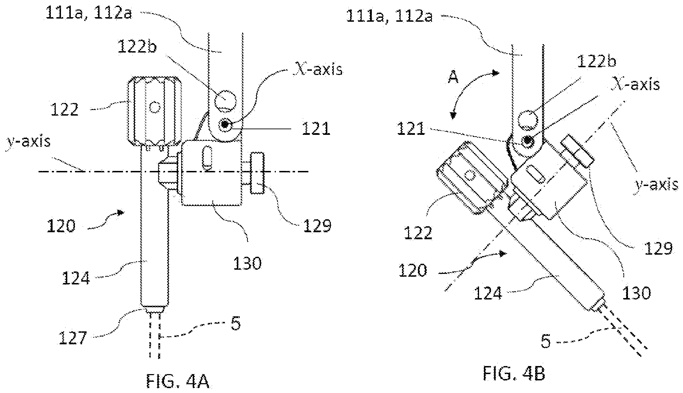

In the orthopedic device **100** according to the present disclosure shown I FIG. 1, locking sleeve **120** attachments are hingeably connected to the outer ends of the first and second arm members **111, 112** by a biaxial hinge block **130** shown in FIGs. 4A-9B. The locking sleeves **120** are configured for receiving and locking on to an elongated pin and the biaxial hinge block **130** is configured to allow each of the locking sleeve to swivel in two different directions with respect to its corresponding arm member, **111** or **112,** about two orthogonally oriented axes. The biaxial hinge block **130** is shown and described in detail below in conjunction with FIGS. 4A through 9B.

In FIG. 1, the orthopedic device **100** is in distraction mode and the locking sleeve attachments **120** holding elongated pins **5** are shown swiveled toward each other forming an angle θ between the two locking sleeve attachments **120.**

Referring to FIGS. 4A through 5C, the orthopedic device **100** also includes a locking sleeve **120** hingeably connected to the outer end of each of the first and second arm members **111, 112** by a biaxial hinge block **130,** wherein each locking sleeve **120** is configured for lockably receiving an elongated pin (not shown).

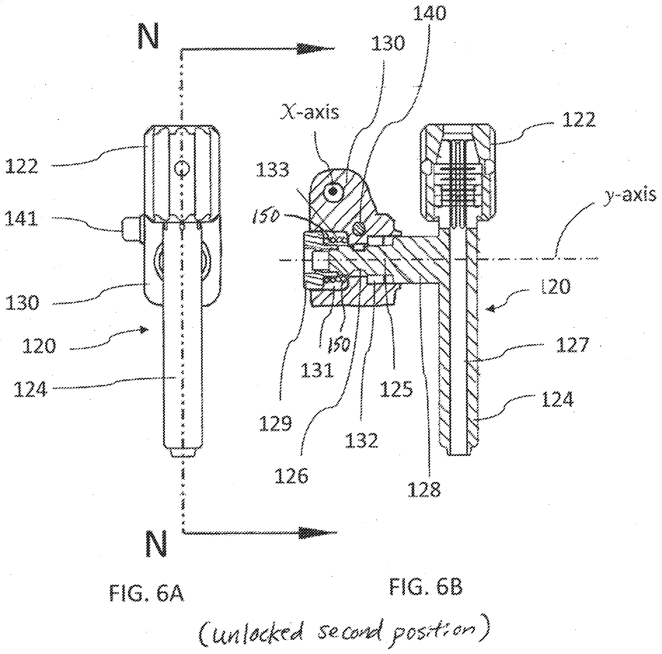

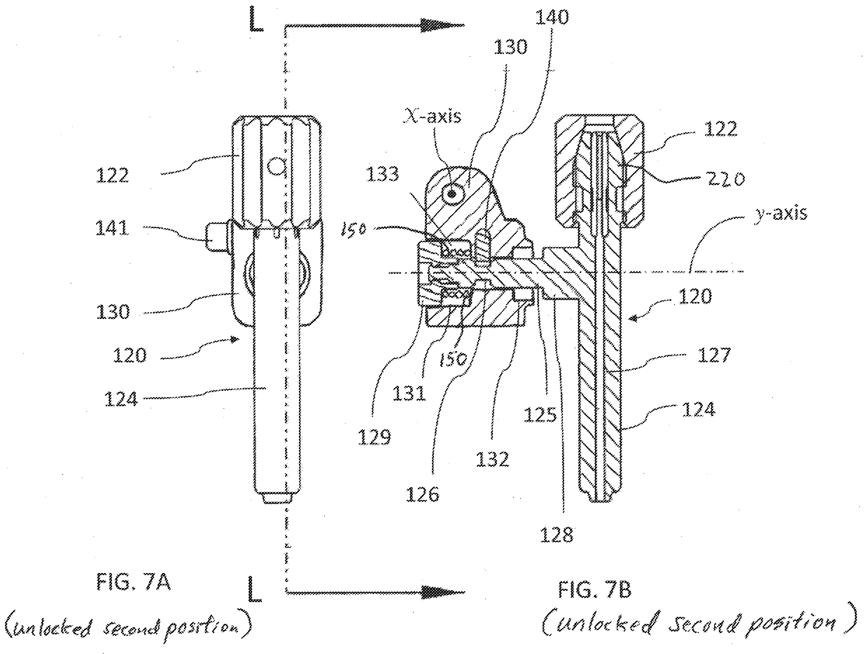

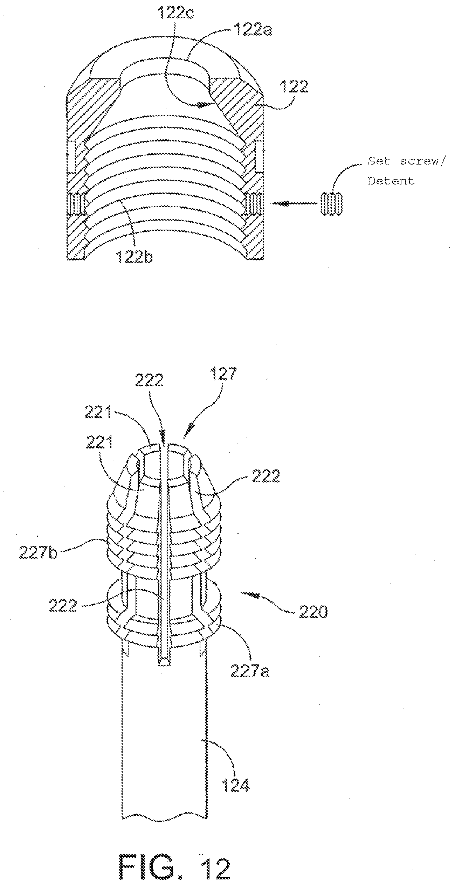

The locking sleeve **120** comprises an elongated shaft **124** having an elongated pin receiving bore **127** (see FIGS. 6B, 7B, 8B, 9B) extending therethrough and a threaded collet **220** (see FIG. 7) provided at one end of the elongated shaft for locking onto or securely holding the elongated pin received in the pin receiving bore **127.** Referring to FIG. 12, the threaded collet **220** comprises a plurality of collet arms **221,** defined by slots **222,** provided with screw threads **227a, 227b** integrally formed on their exterior surfaces, and a collet nut **122** that is threadably engaged to the threaded collet **220** for locking or securely holding the elongated pin **5** that is received in the elongated pin receiving bore **127.**

FIG. 12 shows detailed structures of the threaded collet **220** and the collet nut **122.** As shown in the longitudinal cross-section view of the collet nut **122** in FIG. 12, the collet nut **122** is open at one end for receiving the threaded collet **220** and has an interior surface provided with screw threads **122b** for threadably engaging the screw threads **227a, 227b** of the collet **220.** At the end opposite from the threaded collet receiving end, a through hole **122a** is provided for the elongated pin received in the elongated pin receiving bore **127.** The interior surface of the collet nut **122** is configured with a conical surface **122c** for engaging collet arms **221.** When an elongated pin **5** is received in the elongated pin receiving bore **127,** the threading action of the collet nut **122** causes the collet arms **221** to move radially inward and clamp onto the elongated pin and lock the elongated pin in place. Generally, it is envisioned that the user can lock an elongated pin received in the elongated receiving bore **127** by tightening the collet nut **122** by hand. Further details of the structures of the threaded collet **220** and the collet nut **122** can be found in the United States patent Application Serial No. 13/712,300, filed by the Applicant on December 12, 2012, the contents of which are incorporated herein by reference.

As shown in FIGS. 4A through 5C, each of the biaxial hinge blocks **130** is configured to allow the locking sleeve **120** to swivel in two directions about two orthogonally oriented swivel axes, *x*-axis and *y*-axis. The biaxial hinge blocks **130** can connect the locking sleeves **120** directly to the outer ends of one of the arm members **111, 112** similar to the way the hinge joints in the orthopedic device disclosed in United States patent Application Serial No. 13/712,300 connect the locking sleeves to the outer ends of the arm members **111, 112.** In the embodiment of the present disclosure, the outer ends of the arm members **111, 112** are configured for modular connection of the locking sleeves **120** or some other attachments.

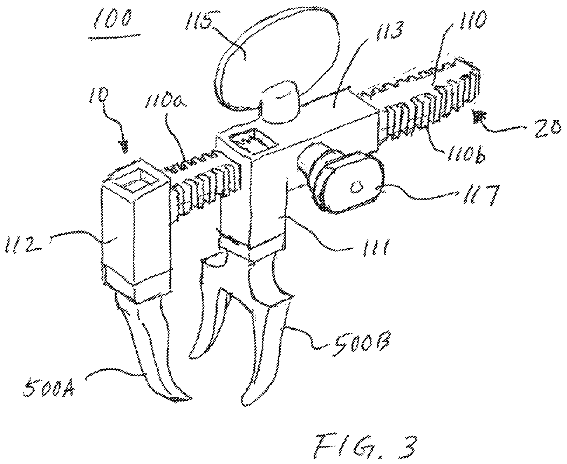

An example of the orthopedic device **100** with 3-point bending yoke attachments **500A** and **500B** attached to the first and second arm members **111, 112** is shown in FIG. 3. It does not fall within the scope of the appended claims. Such 3-point bending yoke attachments can be used to accomplish radial distraction of a bone segment.

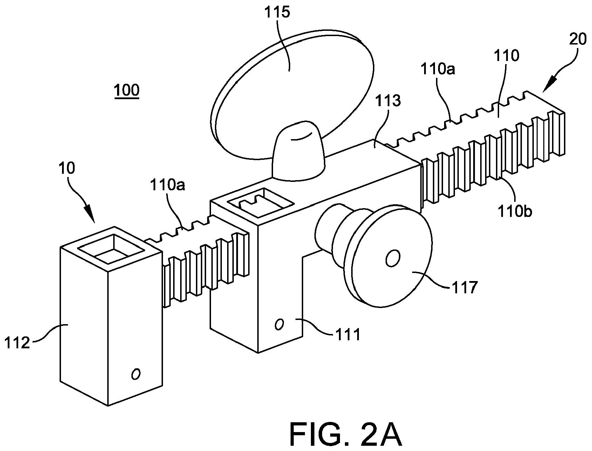

As shown in FIG. 2A, the outer ends of the first and second arm members **111, 112** are configured to receive attachments. As shown in FIG. 5C, the biaxial hinge block **130** is hingeably connected to an arm extension piece **111a, 112a** which is configured to removably attach to the first and second arm members **111, 112.** The specific structures that will enable such attachment between the first and second arm members and the arm extension pieces can be one of a variety of structures that are well known or obvious to one of ordinary skill in the art and need not be described in detail here.

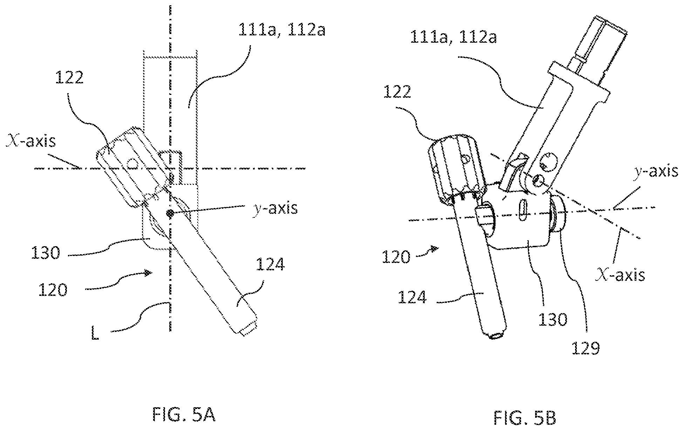

Referring to FIGS. 4A and 4B, the biaxial hinge block **130** includes a first hinge joint that swivels about a first swivel axis, *x*-axis. The first hinge joint allows the locking sleeve **120** to swivel about the *x*-axis in a first direction which is represented by the arrow **A** in FIG. 4B. The first hinge joint can include a swivel pin **121** forming the x-axis and connecting the biaxial hinge block **130** to the arm extension piece **111a, 112a.** The biaxial hinge block **130** also includes a second hinge joint that swivels about a second swivel axis, *y*-axis, that is orthogonal to the first swivel axis. Thus, the biaxial hinge block **130** allows the locking sleeve **120** to swivel in two directions about the two orthogonally oriented swivel axes. FIG. 5A shows the locking sleeve **120** swiveled about the second swivel axis, *y*-axis, so that the locking sleeve **120** is tilted away from the longitudinal axis **L** of the arm extension pieces **111a**, **112a.** FIG. 5B shows an orthogonal projection view of the locking sleeve **120** swiveled about both swivel axes of the biaxial hinge block **130.**

In the illustrated example, the first hinge joint is formed by a pin **20** that is aligned with the *x*-axis and connects the biaxial hinge block **130** to the arm extension pieces **111a, 112a.** The first swivel axis, *x*-axis, is oriented parallel to the elongated body **110** of the orthopedic device.

According to an aspect of the present disclosure, the first hinge joint can be configured and adapted to be normally locked at a desired swivel angle and prevented from swiveling about the first swivel axis, *x*-axis, by a spring-loaded locking pin **112b.** When the spring-loaded locking pin **112b** is pressed, the first hinge joint is unlocked and free to swivel about the *x*-axis.

The second hinge joint of the biaxial hinge block **130** will be described in more detail using the additional FIGS. 6A through 11B. The second hinge joint is formed by a swiveling shaft **125,** that is orthogonally extending from the elongated shaft of the locking sleeve **120** and received in the biaxial hinge block **130** along the second swivel axis, *y*-axis. The swiveling shaft **125** is movable within the biaxial hinge block **130** along the second swivel axis between two positions. A locked first position that keeps the locking sleeve **120** in a non-swiveling position preventing the locking sleeve **120** from swiveling about the second swivel axis, and an unlocked second position allowing the locking sleeve **120** to swivel about the second swivel axis.

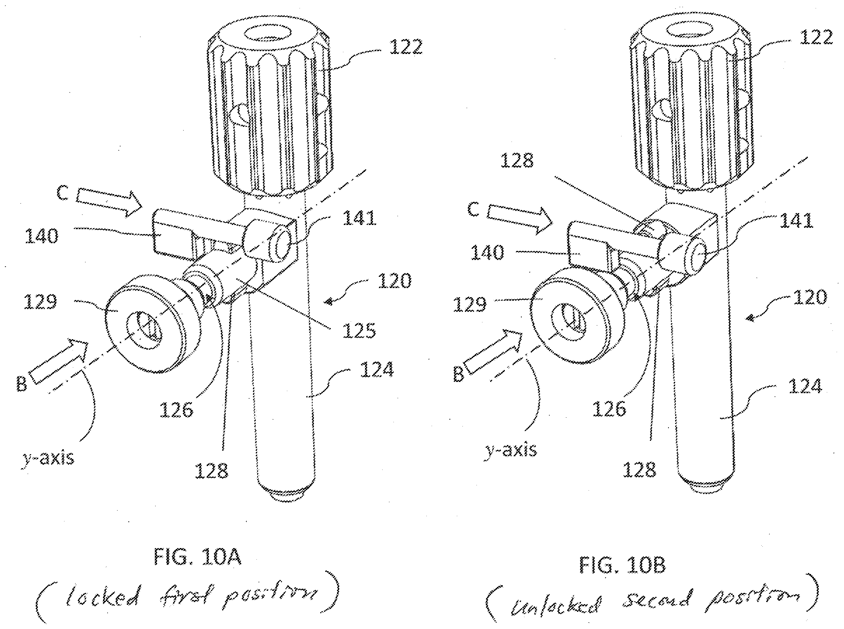

FIGS. 6A - 7B show the swiveling shaft **125** in its unlocked second position within the biaxial hinge block **130,** wherein the locking sleeve attachment **120** can swivel about the *y*-axis. FIG. 6A is an illustration of the locking sleeve attachment **120** connected to the biaxial hinge block **130** viewed along the *y*-axis showing the plane K-K extending through the center of the locking sleeve attachment **120** along which the cross-sectional view of FIG. 6B is taken. FIG. 6B is the cross-sectional view taken along K-K. FIG. 7A is the illustration of FIG. 6A showing the plane L-L extending off-center through the locking sleeve attachment **120** along which the cross-sectional view of FIG. 7B is taken. FIG. 7B is a cross-sectional view along L-L. The biaxial hinge block **130** is provided with a through-hole **131** for receiving the swiveling shaft **125** therethrough. The swiveling shaft **125** has an annular groove **126** and the biaxial hinge block **130** is provided with a locking key **140** that engages the annular groove **126** and keeps the locking sleeve **120** in the unlocked second position. The engaging relationship between the locking key **140** and the swiveling shaft **125** can be better seen in FIG. 10B in which only the locking sleeve's swiveling shaft **125** and the locking key **140** are shown without the biaxial hinge block **130.** The locking key **140** sits within the annular groove **126** and prevents the swiveling shaft **125** from moving along the *y*-axis while allowing the swiveling shaft **125** and, thus, the locking sleeve **120** to swivel about the *y*-axis.

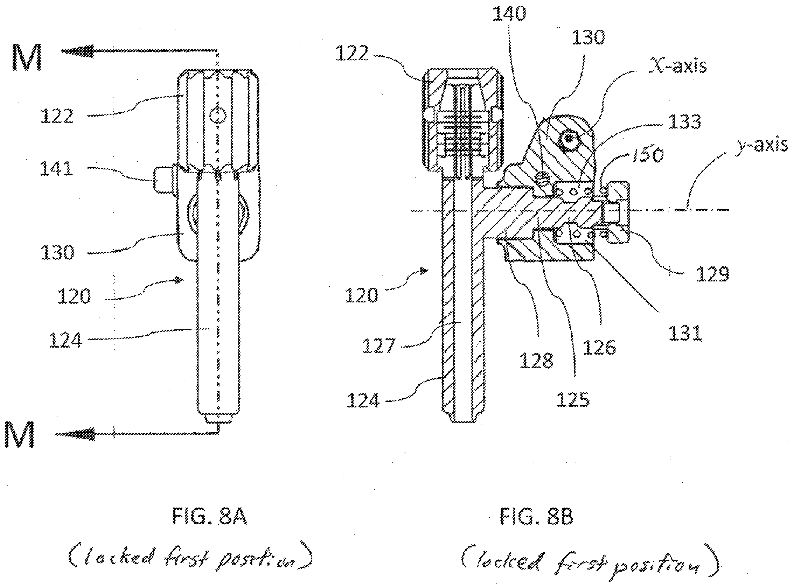

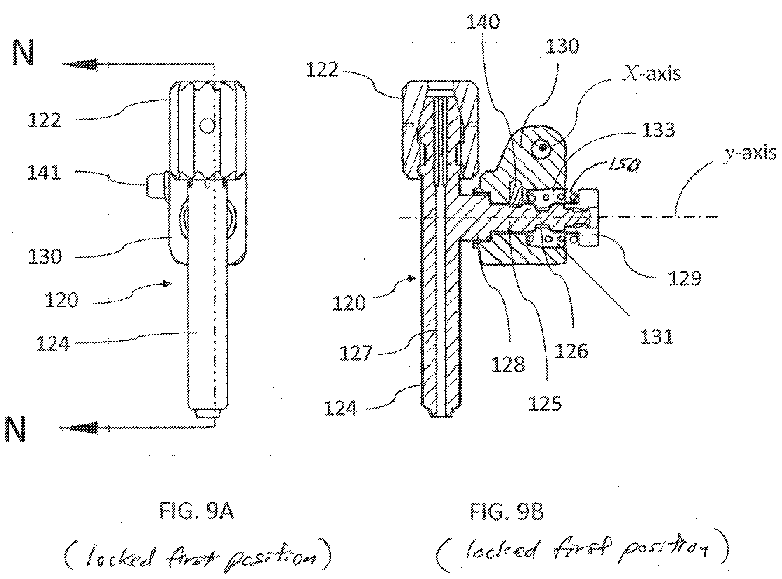

FIGS. 8A - 9B show the swiveling shaft **125** in its locked first position, the non-swiveling position, within the biaxial hinge block **130.** The locking sleeve attachment **120** is in a fixed orientation and cannot swivel about the *y*-axis. FIG. 8A is the same view as FIG. 6A but showing the plane M-M extending through the center of the locking sleeve along which the cross-sectional view of FIG. 8B is taken. FIG. 8B is a cross-sectional view taken along M-M. FIG. 9A is the same view as FIG. 6A but showing the plane N-N extending off-center through the locking sleeve along which the cross-sectional view of FIG. 9B is taken. FIG. 9B is a cross-sectional view taken along N-N. In the locked first position, the swiveling shaft **125** is pushed further into the through-hole **131** of the biaxial hinge block **130** so that the locking key **140** is no longer sitting within the annular groove **126.** Referring to FIGS. 10A - 11B, the swiveling shaft **125** is provided with one or more alignment tabs **128** near the base portion (the part of the elongated shaft **124** connected to the elongated shaft **124)** of the swiveling shaft **125** and the through-hole **131** of the biaxial hinge block **130** has an alignment-tab-receiving end **132** (see FIG. 7B) that is configured and adapted to receive the alignment tabs **128.** Preferably, the alignment tabs **128** are two tabs oppositely located on the base portion of the swiveling shaft but other non-symmetrically positioned arrangements are also contemplated.

The alignment-tab-receiving end **132** of the through-hole **131** has an opening outline that matches the transverse cross-sectional outline of the alignment tabs **128** so that in the locked first position, where the swiveling shaft **125** is pushed further into the through-hole **131,** the alignment tabs **128** engage or mate with the alignment-tab-receiving end **132** and prevent the locking sleeve from swiveling about the *y*-axis.

In one embodiment, the locked first position holds the locking sleeve **120** in an orientation that keeps the elongated shaft **124** of the locking sleeve **120** in a parallel orientation with the arm members **111, 112** of the orthopedic device. In another embodiment, the alignment tabs **128** and the alignment-tab-receiving end **132** can be configured to hold the locking sleeve **120** in any desired angular orientation about the *y-*axis.

Referring to FIGS. 6A-9B, the swiveling shaft **125** is provided with an end cap **129** that captures a coil spring **150** inside an end-cap-receiving portion **133** of the through-hole **131.** The coil spring is in a normally compressed state inside the end-cap-receiving portion **133** so that the coil spring is always urging against the end cap **129** pulling the swiveling shaft **125** further into the through-hole **131** and toward the locked first position shown in FIGS. 8A - 9B.

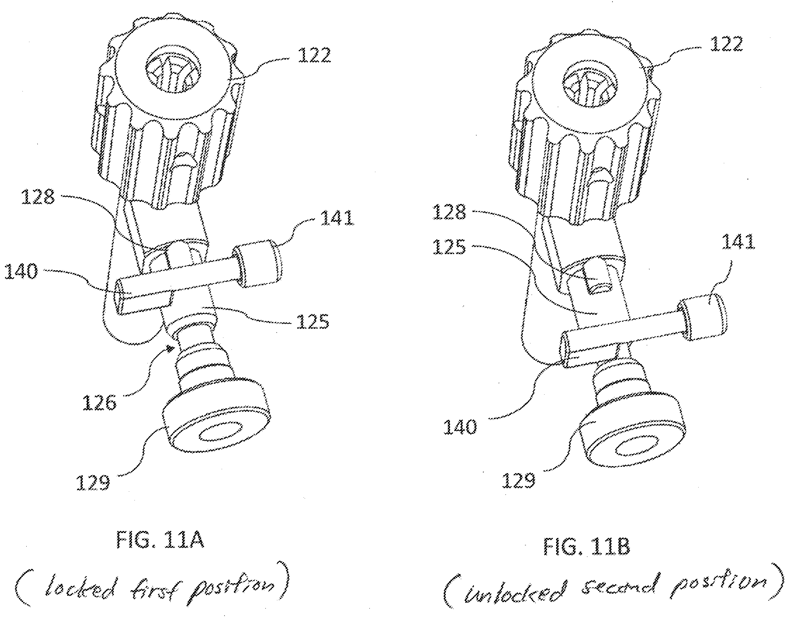

FIGS. 10A and 11A show the positional relationship of the locking key **140** and the swiveling shaft **125** when the swiveling shaft **125** is in the locked first position. In the locked first position, the locking key **140** is not sitting in the annular groove **126.**

According to an embodiment, the locking key **140** is spring-loaded within the biaxial hinge block **130** to be urged in the direction **C** shown in FIGS. 10A and 10B, which is transverse to the *y*-axis, the second swivel axis. To unlock the swiveling shaft **125** from the locked first position shown in FIGS. 9B and 10A, the end cap **129** of the swiveling shaft is pushed in the direction **B** shown in FIG. 10A. This moves the swiveling shaft **125** along the y-axis into its unlocked second position and the annular groove **126** comes in alignment with the locking key **140.** Because the locking key **140** is spring-loaded and being urged in the direction C, the locking key **140** will slide into the annular groove **126** and prevents the swiveling shaft **125** from backing out and keeps the swiveling shaft **125** in its unlocked second position. To move the swiveling shaft **125** back to its non-swiveling first position, the end cap **141** of the locking key **140** is pushed in the direction opposite **C,** thus sliding the locking key **140** out of the annular groove **126.** With the locking key **140** out of the annular groove **126,** the spring-loaded swiveling shaft **125** will slide along the y-axis in direct opposite **B** and return to its first position if he alignment tabs **128** are aligned with the mating outline of the alignment-tab-receiving end **132** of the through-hole **131.**

As described, the orthopedic device **100** of the present disclosure is a universal device that can be used for compression or distraction of bone parts that are secured to the first and second arm members **112, 111** by elongated pins, such as K-wires, locked into the elongated pin receiving bores **127** of the locking sleeves **120.** Referring to FIG. 1, after the bone parts are secured, the movable second arm member **111** can be moved in compression direction **201** or distraction direction **202** by turning the turning key handle **115.**

Although the invention has been described in terms of exemplary embodiments, it is not limited thereto. The scope of the invention disclosed herein is to be limited only by the following claims.

## Claims

1. An orthopedic device comprising:
an elongated body (110) having first and second ends (10, 20);
a first arm member (111) attached to and extending away from said first end and terminating at an outer end;
a second arm member (112) extending from said elongated body and having a base portion and an outer end, wherein the base portion is configured and adapted to engage the elongated body and allow the second arm member to be longitudinally movable along said elongated body, said second arm member extending from the elongated body in the same direction as the first arm member; and
a first locking sleeve (120) hingeably connected to the outer end of the first arm member by a first biaxial hinge block (130),
a second locking sleeve (120) hingeably connected to the outer end of the second arm member by a second biaxial hinge block (130), wherein each locking sleeve is configured for receiving and locking on to an elongated pin and each biaxial hinge block is configured to allow its corresponding locking sleeve to swivel in two different directions with respect to its corresponding arm member about two orthogonally oriented swivel axes, wherein each locking sleeve comprises an elongated shaft (124) having an elongated pin receiving bore (127) extending therethrough and is provided with a swiveling shaft (125) orthogonally extending from the elongated shaft, said locking sleeve connected to the biaxial hinge block by the swiveling shaft being received in the biaxial hinge block along the second swivel axis (y-axis), **characterized in that** the swiveling shaft is movable within the biaxial hinge block along the second swivel axis between two positions, a first position that prevents the locking sleeve from swiveling about the second swivel axis and a second position allowing the locking sleeve to swivel about the second swivel axis.

2. The orthopedic device of claim 1, wherein each biaxial hinge block is provided with a through-hole (131) for receiving the swiveling shaft therethrough, the through-hole having an end-cap-receiving portion, wherein the swiveling shaft is provided with an end cap (129) that captures a coil spring (150) inside the end-cap-receiving portion of the through-hole, wherein the coil spring is in a normally compressed state inside the end-cap-receiving portion urging against the end cap and pulling the swiveling shaft further into the through-hole toward the first position.

3. The orthopedic device of claim 1, wherein the first of the two orthogonally oriented swivel axes is oriented parallel to the elongated body of the orthopedic device.

4. The orthopedic device of claim 1, wherein said locking sleeve further comprises a collet (220) provided at one end of the elongated shaft, said collet comprising a plurality of collet arms (221) provided with screw threads integrally formed on their exterior surfaces, and a collet nut (122) that is threadably engaged to the collet for locking an elongated pin that is received in the elongated pin receiving bore.

5. The orthopedic device of claim 1, wherein the swiveling shaft is provided with one or more alignment tabs (128) near its base portion and the through-hole has an alignment-tab-receiving end that is configured and adapted to receive the alignment tabs, wherein the alignment-tab-receiving end of the through-hole has an opening outline that matches transverse cross-sectional outline of the alignment tabs thereby when in the first position, the alignment tabs engage with the alignment-tab-receiving end and prevent the locking sleeve from swiveling about the second swivel axis.

6. The orthopedic device of claim 1, wherein the first position holds the locking sleeve in an orientation that keeps the elongated shaft of the locking sleeve in a parallel orientation with the arm members of the orthopedic device.

7. The orthopedic device of claim 1, further comprising a locking key (140) provided in the biaxial hinge block that engages the swiveling shaft to lock the swiveling shaft in the second position.

8. The orthopedic device of claim 7, wherein the locking key is spring-loaded within the biaxial hinge block to be urged in the direction transverse to the second swivel axis.

9. The orthopedic device of claim 7, wherein the swiveling shaft is configured with an annular groove (126) that engages the locking key when the swiveling shaft is in the second position.

10. The orthopedic device of claim 9, wherein when the swiveling shaft is in the first position, the locking key and the annular groove are not engaged while the alignment tabs provided on the swiveling shaft and the alignment-tab-receiving end are engaged and thus preventing the swiveling shaft from swiveling in the second swivel axis.

## Patentansprüche

1. Orthopädische Vorrichtung, aufweisend:
einen länglichen Körper (110) mit einem ersten und zweiten Ende (10, 20);
ein erstes Armelement (111), das mit dem ersten Ende verbunden ist und sich von diesem weg erstreckt und an einem äußeren Ende endet;
ein zweites Armelement (112), das sich von dem länglichen Körper aus erstreckt und einen Basisabschnitt und ein äußeres Ende aufweist, wobei der Basisabschnitt dafür ausgelegt und eingerichtet ist, mit dem länglichen Körper in Eingriff zu kommen und dem zweiten Armelement zu ermöglichen, entlang des länglichen Körpers in Längsrichtung beweglich zu sein, wobei sich das zweite Armelement von dem länglichen Körper in dieselbe Richtung erstreckt wie das erste Armelement; und
eine erste Verriegelungshülse (120), die mit dem äußeren Ende des ersten Armelements um einen ersten zweiachsigen Gelenkblock (130) schwenkbar verbunden ist,
eine zweite Verriegelungshülse (120), die mit dem äußeren Ende des zweiten Armelements um einen zweiten zweiachsigen Gelenkblock (130) schwenkbar verbunden ist, wobei jede Verriegelungshülse dafür ausgelegt ist, einen länglichen Zapfen aufzunehmen und daran einzurasten, und jeder zweiachsige Gelenkblock so gebaut ist, dass seiner entsprechenden Verriegelungshülse ermöglicht ist, in Bezug auf ihr entsprechendes Armelement in zwei unterschiedliche Richtungen um zwei zueinander senkrecht ausgerichtete Schwenkachsen zu schwenken,
wobei jede Verriegelungshülse eine längliche Welle (124) mit einer länglichen Zapfenaufnahmebohrung (127) aufweist, die sich durch sie hindurch erstreckt und mit einer Schwenkwelle (125) versehen ist, die sich von der länglichen Welle aus senkrecht erstreckt, wobei die Verriegelungshülse, die durch die Schwenkwelle mit dem zweiachsigen Gelenkblock verbunden ist, entlang der zweiten Schwenkachse (y-Achse) in dem zweiachsigen Gelenkblock aufgenommen ist,
**dadurch gekennzeichnet, dass**
sich die Schwenkwelle im Inneren des zweiachsigen Gelenkblocks entlang der zweiten Schwenkachse zwischen zwei Positionen bewegen lässt, wobei eine erste Position verhindert, dass die Verriegelungshülse um die zweite Schwenkachse schwenkt, und eine zweite Position der Verriegelungshülse erlaubt, um die zweite Schwenkachse zu schwenken.

2. Orthopädische Vorrichtung nach Anspruch 1,
wobei jeder zweiachsige Gelenkblock mit einem Durchgangsloch (131) ausgebildet ist, um die Schwenkwelle dorthindurch aufzunehmen, wobei das Durchgangsloch einen Abschlusskappenaufnahmeabschnitt aufweist, wobei die Schwenkwelle mit einer Abschlusskappe (129) versehen ist, die im Innern des Abschlusskappenaufnahmeabschnitts des Durchgangslochs eine Schraubenfeder (150) erfasst, wobei sich die Schraubenfeder im Innern des Abschlusskappenaufnahmeabschnitts in einem normalerweise zusammengedrückten Zustand befindet, in dem sie gegen die Abschlusskappe drückt und die Schwenkwelle weiter in das Durchgangsloch in Richtung der ersten Position hineinzieht.

3. Orthopädische Vorrichtung nach Anspruch 1, wobei die erste der beiden zueinander senkrecht ausgerichteten Schwenkachsen zu dem länglichen Körper der orthopädischen Vorrichtung parallel ausgerichtet ist.

4. Orthopädische Vorrichtung nach Anspruch 1, wobei die Verriegelungshülse ferner eine Klemmhülse (220) aufweist, die an einem Ende der länglichen Welle vorgesehen ist, wobei die Klemmhülse mehrere Klemmhülsenarme (221), die mit Schraubengewinden versehen sind, die auf deren Außenflächen integral ausgebildet sind, und eine Klemmhülsenmutter (122) aufweist, die mit der Klemmhülse in Gewindeeingriff gebracht ist, um einen länglichen Zapfen zu verriegeln, der in der länglichen Zapfenaufnahmebohrung aufgenommen ist.

5. Orthopädische Vorrichtung nach Anspruch 1, wobei die Schwenkwelle in der Nähe ihres Basisabschnitts mit einer oder mehreren Ausrichtungsnasen (128) versehen ist, und das Durchgangsloch ein Ausrichtungsnasenaufnahmeende aufweist, das dafür ausgelegt und eingerichtet ist, die Ausrichtungsnasen aufzunehmen, wobei das Ausrichtungsnasenaufnahmeende des Durchgangslochs einen Öffnungsumriss aufweist, der zu einem transversalen Querschnittsumriss der Ausrichtungsnasen passt, so dass dadurch die Ausrichtungsnasen, wenn sie sich in der ersten Stellung befinden, mit dem Ausrichtungsnasenaufnahmeende in Eingriff kommen und ein Schwenken der Verriegelungshülse um die zweite Schwenkachse verhindern.

6. Orthopädische Vorrichtung nach Anspruch 1, wobei die erste Stellung die Verriegelungshülse in einer Ausrichtung hält, die die längliche Welle der Verriegelungshülse in einer parallelen Ausrichtung mit den Armelementen der orthopädischen Vorrichtung hält.

7. Orthopädische Vorrichtung nach Anspruch 1, ferner mit einem Sicherungsstift (140), der in dem zweiachsigen Gelenkblock vorgesehen ist, der mit der Schwenkwelle in Eingriff kommt, um die Schwenkwelle in der zweiten Stellung zu verriegeln.

8. Orthopädische Vorrichtung nach Anspruch 7, wobei der Sicherungsstift im Inneren des zweiachsigen Gelenkblocks federbelastet ist, um in der quer zu der zweiten Schwenkachse verlaufenden Richtung gedrückt zu werden.

9. Orthopädische Vorrichtung nach Anspruch 7, wobei die Schwenkwelle mit einer Ringnut (126) ausgebildet ist, die mit dem Sicherungsstift in Eingriff steht, wenn sich die Schwenkwelle in der zweiten Stellung befindet.

10. Orthopädische Vorrichtung nach Anspruch 9, wobei sich der Sicherungsstift und die Ringnut, wenn sich die Schwenkwelle in der ersten Stellung befindet, nicht im Eingriff befinden, während sich die Ausrichtungsnasen, die an der Schwenkwelle vorgesehen sind, und das Ausrichtungsnasenaufnahmeende in Eingriff befinden, so dass dadurch ein Schwenken der Schwenkwelle in der zweiten Schwenkachse verhindert wird.

## Revendications

1. Dispositif orthopédique comprenant :
un corps allongé (110) ayant des première et seconde extrémités (10, 20) ;
un premier élément de bras (111) fixé à et s'étendant à distance de ladite première extrémité et se terminant au niveau d'une extrémité externe ;
un second élément de bras (112) s'étendant à partir dudit corps allongé et ayant une partie de base et une extrémité externe, dans lequel la partie de base est configurée et adaptée pour mettre en prise le corps allongé et permettre au second élément de bras d'être longitudinalement mobile le long dudit corps allongé, ledit second élément de bras s'étendant à partir du corps allongé dans la même direction que le premier élément de bras ; et
un premier manchon de verrouillage (120) raccordé de manière articulée à l'extrémité externe du premier élément de bras par un premier bloc de charnière biaxial (130),
un second manchon de verrouillage (120) raccordé de manière articulée à l'extrémité externe du second élément de bras par un second bloc de charnière biaxial (130), dans lequel chaque manchon de verrouillage est configuré pour recevoir et se verrouiller sur une broche allongée et chaque bloc de charnière biaxial est configuré pour permettre à son manchon de verrouillage correspondant de pivoter dans deux directions différentes par rapport à son élément de bras correspondant autour de deux axes de pivot orientés de manière orthogonale,
dans lequel chaque manchon de verrouillage comprend un arbre allongé (124) ayant un alésage de réception de broche allongée (127) s'étendant à travers ce dernier et est prévu avec un arbre de pivotement (125) s'étendant de manière orthogonale à partir de l'arbre allongé, ledit manchon de verrouillage étant raccordé au bloc de charnière biaxial par l'arbre de pivotement qui est reçu dans le bloc de charnière biaxial le long du second axe de pivot (axe y),
**caractérisé en ce que** :
l'arbre de pivotement est mobile dans le bloc de charnière biaxial le long du second axe de pivot entre deux positions, une première position qui empêche le manchon de verrouillage de pivoter autour du second axe de pivot et une seconde position permettant au manchon de verrouillage de pivoter autour du second axe de pivot.

2. Dispositif orthopédique selon la revendication 1, dans lequel chaque bloc de charnière biaxial est prévu avec un trou débouchant (131) pour recevoir l'arbre de pivotement à travers ce dernier, le trou débouchant ayant une partie de réception de capuchon d'extrémité, dans lequel l'arbre de pivotement est prévu avec un capuchon d'extrémité (129) qui capture un ressort hélicoïdal (150) à l'intérieur de la partie de réception de capuchon d'extrémité du trou débouchant, dans lequel le ressort hélicoïdal est dans un état normalement comprimé à l'intérieur de la partie de réception de capuchon d'extrémité poussant contre le bouchon d'extrémité et tirant l'arbre de pivotement davantage dans le trou débouchant vers la première position.

3. Dispositif orthopédique selon la revendication 1, dans lequel le premier des deux axes de pivot orientés de manière orthogonale, est orienté parallèlement au corps allongé du dispositif orthopédique.

4. Dispositif orthopédique selon la revendication 1, dans lequel ledit manchon de verrouillage comprend en outre :
une pince (220) prévue au niveau d'une extrémité de l'arbre allongé, ladite pince comprenant une pluralité de bras de pince (221) prévus avec des filetages de vis formés de manière solidaire sur leurs surfaces extérieures, et un écrou de pince (122) qui est mis en prise, par filetage, sur la pince pour verrouiller une broche allongée qui est reçue dans l'alésage de réception de broche allongée.

5. Dispositif orthopédique selon la revendication 1, dans lequel l'arbre de pivotement est prévu avec une ou plusieurs languettes d'alignement (128) à proximité de sa partie de base et le trou débouchant a une extrémité de réception de languette d'alignement qui est configurée et adaptée pour recevoir les languettes d'alignement, dans lequel l'extrémité de réception de languette d'alignement du trou débouchant a un contour d'ouverture qui correspond au contour transversal des languettes d'alignement, ainsi, lorsqu'elles sont dans la première position, les languettes d'alignement se mettent en prise avec l'extrémité de réception de languette d'alignement et empêchent de manchon de verrouillage de pivoter autour du second axe de pivot.

6. Dispositif orthopédique selon la revendication 1, dans lequel la première position maintient le manchon de verrouillage dans une orientation qui garde l'arbre allongé du manchon de verrouillage dans une orientation parallèle avec des éléments de bras du dispositif orthopédique.

7. Dispositif orthopédique selon la revendication 1, comprenant en outre une clé de verrouillage (140) prévue dans le bloc de charnière biaxial qui met en prise l'arbre de pivotement pour verrouiller l'arbre de pivotement dans la seconde position.

8. Dispositif orthopédique selon la revendication 7, dans lequel la clé de verrouillage est à ressort, à l'intérieur du bloc de charnière biaxial pour être poussée dans la direction transversale au second axe de pivot.

9. Dispositif orthopédique selon la revendication 7, dans lequel l'arbre de pivotement est configuré avec une rainure annulaire (126) qui met en prise la clé de verrouillage lorsque l'arbre de pivotement est dans la seconde position.

10. Dispositif orthopédique selon la revendication 9, dans lequel, lorsque l'arbre de pivotement est dans la première position, la clé de verrouillage et la rainure annulaire ne sont pas mis en prise alors que les languettes d'alignement prévues sur l'arbre de pivotement et l'extrémité de réception de languette d'alignement sont mises en prise et empêchent ainsi l'arbre de pivotement de pivoter dans le second axe de pivot.
